# EUROPEAN PATENT SPECIFICATION

(11) **EP 3 753 601 B1**
(45) Date of publication and mention of the grant of the patent: **18.09.2024**
(21) Application number: 19181716.2
(22) Date of filing: 21.06.2019
(51) Int. Cl.: A61M 37/00, A61F 9/00, A61M 35/00

(54) **NEEDLE IMPROVEMENTS FOR DRUG DELIVERY USING TATTOO MACHINES**
NADELVERBESSERUNGEN ZUR ARZNEIMITTELABGABE MIT TÄTOWIERMASCHINEN
AMÉLIORATIONS D'AIGUILLE POUR L'ADMINISTRATION DE MÉDICAMENTS À L'AIDE DE MACHINES DE TATOUAGE

(43) Date of publication of application: 23.12.2020
(73) Proprietor: Trigo Arbache, Samia, 12.246-010 Cit of São José dos Campos, São Paulo (BR)
(72) Inventor: Trigo Arbache, Samia, 12.246-010 Cit of São José dos Campos, São Paulo (BR)
(74) Representative: Herrero & Asociados, S.L.

(56) References cited:
- BR-A2- 102015 020 843
- CN-U- 207 462 458
- US-A1- 2007 260 201
- US-A1- 2014 271 897
- US-A1- 2014 316 326

## Description

### TECHNICAL FIELD OF INVENTION

This invention privilege patent belongs to the field of medicine and surgical instruments, and is intended to provide a new route of drug administration through needles plated with gold, aiming at better drug distribution and absorption upon administration, more precisely in the eyeball and/or on the patient's skin.

To which the product has been originally improved in order to increase its efficacy in relation to other means of drug delivery known in the medical field, especially in skin, mucous and ocular diseases.

It is an improvement that allows drug delivery to the skin and/or subcutaneous tissue, by means of a duly modified tattoo machine, which instead of ink, uses drugs, as a product to be inserted into the skin. In addition, this invention can be used to administer drugs directly to a person's eyeball, offering great practicality and agility to surgical procedures.

Also, the purpose of this application is to present improvements made to needles for drug delivery using tattoo machines, with low industrial manufacturing costs, combined with requirements of effectiveness, efficiency and safety, providing an additional treatment option which, unlike the usual methods, offers numerous possibilities and benefits to its users, making it a product of great acceptance in the medical sector.

### BACKGROUND OF THE INVENTION

The oldest invasive route to administer active ingredients to the body is probably the technique used to tattoo the skin. There is great archaeological evidence that tattoos were already done in Egypt, between 4,000 and 2,000 BC, and also by Polynesian, Philippine, Indonesian and New Zealand natives.

In 1991, Otzi mummy was found in the Alps. It was a well-preserved male corpse, about 5,300 years old.

Otzi had 61 (sixty-one) tattoos coincidentally located on acupuncture points. Anthropologists believe that they probably had some therapeutic purpose.

Tattoos can be performed by various instruments. From iatrogenic tattoos (secondary to skin injection, mainly iron-based drugs), accidental tattoos (secondary to skin abrasion by asphalt), tattoos performed by solid needles or pointed instruments (known as amateur tattoos) and tattoos performed by machines (known as professional tattoos).

Tattoo machines are highly effective in injecting ink to the skin. This 1,000-year old technique gives machine operators an interesting route for administering ink to the skin.

The eye is a highly sophisticated and delicate structure. Simply stated, it is a device that resembles a camera, capturing static or moving images of the surroundings and transmitting them through the optic nerve to the central nervous system to be identified and interpreted. However, its sophistication contrasts with the inability of physicians to permeate its structures with drugs, fundamental to treat eye diseases. Drug bioavailability by transcorneal (through the cornea) and transconjunctival absorption is very low (e.g. eye drops), and the necessary therapeutic levels for eye disease treatment are not achieved.

Treatment of inflammatory, infectious and degenerative eye diseases are still a therapeutic challenge today. Oral, systemic, or topical drugs (eye drops) are unable to reach therapeutic doses at sites where there is infection or active inflammation, which makes current ophthalmological treatments ineffective in controlling infectious, inflammatory, and degenerative keratitis (inflammation of the cornea). Hence, the morbidity (number for patients affected by a particular disease) of ophthalmological diseases is high, forcing ophthalmologists to perform surgical interventions with high costs for the healthcare system (e.g. penetrating corneal transplants). The social and economic impact of "incurable" eye diseases is significant, occasionally leading to tragic outcomes such as blindness, in some cases evolving to evisceration (surgical removal of the eyeball) by purulent endophthalmitis (eye infection) secondary to keratitis (inflammation of the cornea). Conjunctival and scleral disorders are another therapeutic challenge, since both are highly immunoreactive means, sites of hypersensitivity reactions difficult to be clinically controlled, and autoimmune diseases affecting the vascular system with high morbidity levels.

Therefore, in this scenario, designing techniques to easily inject drugs to the eyeball is fundamental.

Drug injections to the eye using a syringe is a common method employed by ophthalmologists, the most commonly performed routes being intracorneal (corneal injections) or intraocular (injections into the eye).

Intracorneal and intraocular injections with syringes are valid procedures accepted by the global scientific community because they effectively inject drugs into the eyeball. However, these procedures are technically dependent because they are subject to physician's finger pressure variability when pressing the syringe plunger, with consequent irregular distribution of the drug in the area to be treated, with the possibility of sequelae. If the pressure exerted on the syringe is exaggerated, it may cause irregular distribution of the drug and adverse effects by a compressive effect.

In summary, ophthalmologists have technical limitations to treat the eyeball with drugs.

By retrospectively reviewing the topic, we have identified in the literature references to ocular interventions performed by Galen (150 AD) and Aetius (450 AD) intended to pigment-cover corneas with leukoma (white opacity of the cornea that resembles scars and blindness, usually secondary to ulcers or corneal trauma) using inks similar to the ones used for tattooing.

The corneal pigmentation technique was again performed in 1870 by the oculoplastic surgeon Louis Von Wecker, laying the foundations of the modern micro pigmentation technique for corneal scars in eyes without sight, widely known in current medicine, being improved for the use of multiple needles and different pigments, as in the series of publications by Archer et al (Utrecht, 1874).

Until recently, corneal dermopigmentation was indicated for corneal leukomas only in eyes whose visual prognosis was extremely reserved, especially in patients who did not adapt to the use of cosmetic contact lens. In these cases, inserting pigments through needles to cover disfiguring leukomas was quite successful.

Recently, an equipment has been described that impact-inserts a tiny biodegradable tip into the cornea that gradually releases drug. The tip of this device is pre-formed with the drug and inserted under pressure at the base of the needle.

Another technique includes introducing drug-coated needles, always with the same purpose, to deliver drugs to the cornea.

Aiming at introducing a new product to the market, a state of the art search was carried out in a specialized database and the following processes were found:
PI 9900009-1 - "Transfer device of at least one active product inside the human eyeball by iontophoresis", this invention relates to a transfer device of at least one active product, specifically a drug, to the eyeball by means of iontophoresis, comprising an active product reservoir (15), and capable of being applied over the eye of a patient. The reservoir (15) has at least one surface active electrode (11) placed across an eye tissue located at the periphery of the cornea (C), a return electrode (12) and a current generator. The return electrode (12) is preferably in contact with the patient's partially closed eyelids (22, 24).

BR 11 2015 025358-0 - "Eye dropper and method for administering liquid drops to eye surface", an eye dropper (2, 102) with improved stability is provided for delivering a volume of liquid on the surface of the eye (57). The eye dropper (2, 102) includes a body (4, 104) having a reservoir body (18, 118), a first rod (14, 114), a second rod (16, 116), and a mouthpiece (26, 126). The mouthpiece (26, 126) is in fluid communication with the inner cavity (24, 124) of the reservoir body (18, 118) and is projected from the outer surface (20, 120) of the reservoir body (18, 118) between the first and second rods (14, 16, 114, 116). The first and second rods (14, 16, 114, 116) are separated by a distance which is at least equal to the height of the eye dropper (2, 102). In preferred embodiments, the distance between the first and second rod (14, 16, 114, 116) is greater than the height of the reservoir body (18, 118).

BR 11 2017 008658-1 - "Syringe and syringe preparation method", the present invention relates to a syringe (18) having a longitudinal body (28) with an interior (228) containing a pharmaceutical substance, a needle connected to a longitudinal end of the body (228) and a rigid needle guard (38) surrounding the needle. The rigid needle guard (38) is essentially impermeable to water vapor. The syringe (18), according to the invention, prevents needle clogging and, thus, allows the adequate delivery of pharmaceuticals, particularly by subcutaneous, intramuscular or ocular injection.

BR 11 2015 027762-4 - "Ocular injection apparatus and methods", it is an apparatus including a housing coupled to a drug container which is coupled to a needle. An injection assembly is located within the housing and includes a power storage member and actuating rod. A distal end portion of the actuating rod is located inside the drug container. The energy storage member may produce a force on a proximal end portion of the actuating rod sufficient to move the distal end portion of the actuating rod inside the drug container. This may carry at least a portion of a substance from the drug container through the needle when a distal tip of the needle is located within a first region of a target location. The force is insufficient to move the distal end portion of the actuating rod inside the drug container when the distal tip of the needle is located within a second region of the target location.

In addition, various previous state of the technique are known which are related to ink infusion on the skin, nothing specific for drug infusion, the identifications and summaries of which are listed below.

US 6505530 B2 - "Ink delivery device for tattooing or permanent makeup", an ink application device for tattooing or permanent makeup that includes a handle, a needle unit, a needle, a needle tip and a protective case. The device further comprises at least two modules connected to one another. One of the modules is formed as a reusable base module with an integrated needle unit, and the other module is a sterile disposable module in which all components of the handheld device can be infected by a patient's body fluids. The device can have its construct divided in two or three distinct modules: a basic module, a disposable or hygienic module, and an optional ink module. The base module corresponds to the handle which includes the needle unit and is designed so that the hygiene module can be linked and connected securely to the integrated needle unit.

US 20050010236 A1 - "Ink device for tattooing or permanent makeup", the invention relates to an apparatus used for permanent tattooing or a composition, comprising a disposable detachable module arranged in a handpiece, a needle held in a end member on a needle shaft and supported for movement of a needle guide formed in the disposable module and the other end extending through an opening of the discharging mouthpiece to the color needle, and a drive mechanism comprised of several structural elements and coupled to the needle axis to effect needle back and forth movements, said plurality of structural elements of the drive mechanism of the unit and that comprises means for coupling the drive devices to the needle rod. At least a portion of the coupling means is located in a disposable and detachable module of the handpiece together with the disposable module.

US 20100036317 A - "Portable device for human or animal skin puncturing site, drive unit, needle unit and a coupling method", the invention relates to a portable device for a human or an animal skin puncture site, in particular for the introduction of an active substance or for the application of a permanent tattoo or of a composition comprising a drive unit, set up to move the repetitive unit, and a needle unit, which comprises a needle device and is set up to be coupled with the drive unit so that movement of the repetitive unit for needle extension and retraction in the device can be coupled to the latter; said drive unit has an actuation member on it, which is functionally displaceable, coupled to a needle attachment device, and said needle unit having therein a functional member that is assigned to the drive member, said drive member and said functional member are set up to move the drive member from a first displacement position to a second displacement position by means of the functional attachment member designated as the needle unit for the drive unit, thus moving the needle attachment device to an engaging position in which the needle can be coupled to the needle attachment device for the purpose of operation. Further, the invention relates to a method for coupling a needle unit to a drive unit of a hand device, a drive unit for a hand device, as well as a needle unit for a portable device.

US 7340980 B2 - "Tattoo machine", a tattoo machine comprising at least one elongated hollow body, within which there is a cavity for containing a staining liquid connected to the outside by an orifice at one of the ends of the hollow body, a needle that can slide alternatively along the holes, and elements for the passage of the needle, between a first end position, where the needle projects at least partially from the hollow body, and a second end position, where the needle is fully accommodated within the hollow body, the elements of the movement comprising a motor which can be associated with the needle, with interposition of movement transmission elements supplied with a flexible cable, at a first end of which may be associated with the motor and a second end that may be associated with the needle.

US 20050277973 A - "Ultrasonic tattooing equipment", an ultrasound device for applying skin pigmentation in which the device moves the needle to apply the pigment in an ultrasound band. The device may be self-contained, self-powered, or powered externally. The invention may also include replaceable needles, and/or a pigment reservoir. The device may also have course length adjustments to prevent stroke. The mechanism that moves the needle can be a piezo unit, a linear unit or a mechanism of solenoid type or an actuator unit.

US 20120221036 A - "Tattooing device", a method and apparatus for marking a treatment isocenter in a patient's body. One embodiment includes a base, including alignment marks, a marker disposed on the base and positioned relative to the isocenter marking reference on the patient's body and an actuator for triggering the marker and causing a mark indicating the isocenter in the patient's body. The actuator may include a key and a spring attached to the marker. Actuator compressing causes the marker to pass through an ink well before piercing the patient's skin.

The major problem of skin treatment methods using topical drugs, on the one hand, has to do with the body having difficulty absorbing topical drugs, since the skin, as a human organ, is a natural protection barrier that prevents the desired drug absorption. On the other hand, injectable skin treatment methods deliver excessive, irregular and/or deficient amounts of drug at a single point, making absorption disproportionate as to the amount of drug to be absorbed by the total area of the skin to be treated.

A high-frequency fractionating device called "LEGATTO" has been designed, which proposes a solution similar to the method in the presentation "to perfuse" drugs on the skin, but it is a completely different technique in which radio-frequency equipment makes micro perforations on the skin, delivers the drug to the skin and subsequently applies an ultrasound handpiece to force absorption.

Document CN207462458U provides an insert look embroidery needle. The one end of well hollow needle pipe is the syringe needle, the medial surface of fixed plate divides pin arranging the tube seat, the needle tubing trench of first fixed plate is put to put with the needle tubing trench of second fixed plate and is corresponded, the needle tubing groove is half circular, individual needle tubing flute profile becomes round, the outer circumference contact of round and well hollow needle pipe, there are sealing washer and well hollow needle union coupling, the other end of well hollow needle pipe passes through the sealing washer and connects in the needle tubing groove of fixed plate, the one end of pressure strip is sealed, the other end of pressure strip has first screw and first knob nut fixed joint, hollow needle pipe and fixed plate is connected in compressing tightly.

Document US2014316326 relates to an injection device comprising a first support having a cup-shaped first contact surface intended to come into contact with a first region of an outside surface of an eye, and a set of at least four injection needles in fluid communication with each other and protruding from said first contact surface at respective insertion points so that the distance between the distal end of any of said injection needles to said first contact surface is between 0.6 mm and 1.3 mm. The insertion points of the injection needles on the first contact surface are spread on said first contact surface so that the diameter of the largest circle that it is possible to include completely in the convex surface defined by said insertion points on a front view of said first contact surface, without any insertion point being included in said circle, is less than 8 mm.

Document BR102015020843A2 discloses a method for microinfusion of medicines into the skin using a tattoo machine. Document US2007260201 provides methods and devices for administering a drug to a patient's eye. The methods include (a) inserting a hollow microneedle into the sclera or corneal stroma without penetrating across the sclera or corneal stroma; and (b) infusing a fluid drug formulation through the microneedle and into the sclera or cornea. It further may include partially retracting the microneedle before infusion to enhance delivery. Finally, document US2014271897 describes compositions, methods and devices for sustained drug delivery. The microencapsulated sustained drug delivery compositions are deposited using oscillating needle apparatus oscillating at 10-12000 minutes per minutes. The injected compositions may undergo variety of physical chemical changes upon deposition. The physical and chemical changes enable improved drug encapsulation and sustained drug release.

It is found that the above-mentioned documents and the methods described in the prior art, although belonging to the same field, do not have the same technical characteristics of the object hereby improved, thus ensuring it meets the necessary legal requirements for patentability.

### BRIEF DESCRIPTION OF THE INVENTION

The invention is defined in independent claim 1.

In order to overcome the drawbacks and limitations present in the state of the art, the inventor has created and developed the present invention by devising needles for injecting drugs into the corneal stroma and/or into eye structures and into the dermis or subcutaneous tissue through tattoo machines, in which the needles make a back and forth movement.

Tattooing, that is, the insertion of chemicals into the skin by means of needles, is a 1,000-year old technique. Eye tattooing was described by Galen in 150 AD and forgotten by humankind. Ocular pigmentation was rediscovered by De Wecker, fourteen centuries later. Afterwards, several studies described drug delivery into the eyeball using needles impregnated with drugs.

Backed up by the history of tattoos documented thousands of years ago and by the scientific documentation researched, pigments and drugs were injected into the eyes of rabbits for testing purposes after ethical approval from an Animal Ethics Committee. Subsequently, after ink and drug delivery, anatomical and pathological examinations confirmed that the injection of chemicals into the eyeball is an effective procedure, without any adverse effect being detected.

The main advantage of injecting drugs into the eyeball using tattoo machines with specific needles is that it is possible to inject drugs in small quantities with homogeneity that cannot be achieved when the same procedure is performed by a physician using syringes and needles, since application is irregular and technically-dependent.

In addition, the present patent has also been developed on the basis of proven drug skin absorption research which has resulted in the adaptation of a tattoo machine, conveniently configured and arranged to allow drug delivery to be better distributed under the skin, with unparalleled effectiveness and versatility, without the drawbacks already mentioned.

In existing needle models already manufactured with the drug inserted into the needle, whether in its structure or by coating, physicians are not able to choose the drug and its strength.

In the technique described herein, physicians can choose specific drugs for the eye/skin disease and also decrease or increase the strength of the drug to be injected.

It is understood that the present invention developed for the application of the technique in question offers excellent results, thus incorporating an innovative method that makes it possible to minimize potential inconveniences.

### DESCRIPTION OF THE FIGURES

To complement the present description in order to obtain a better understanding of the features of the present invention and in accordance with a preferred practical embodiment thereof, a set of figures, attached, accompany the description, where operation has been represented with examples that are not limited:
Fig. 1: shows the detail of needles with tips aligned with the curvature of the eye;
Fig. 2: shows the detail of needle tip oval arrangement;
Fig. 3: shows the detail of the needles seen from below;
Fig. 4: shows a perspective view of the needles;

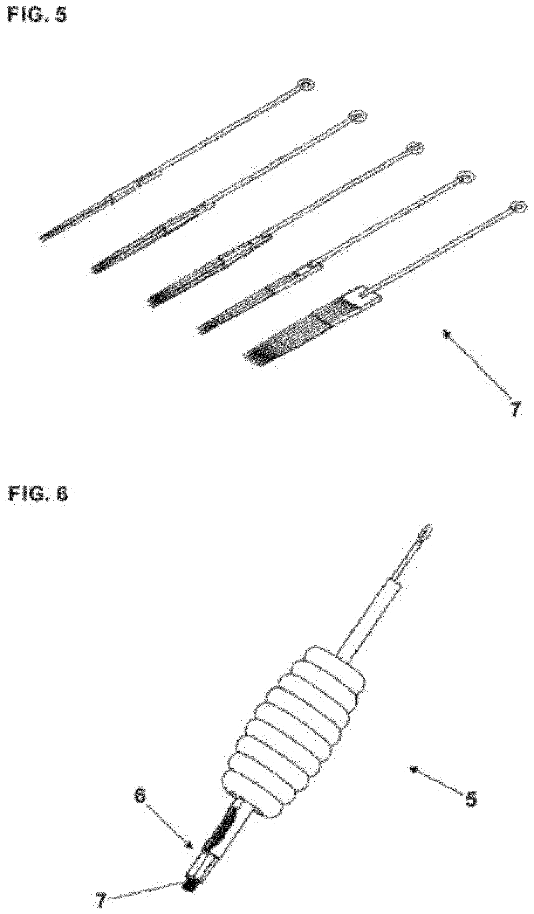

Fig. 5: shows a perspective view of a set of tattoo needles used in the method for drug micro infusion on the skin through a tattoo machine.
Fig. 6: shows a perspective view of a tattoo tip used in drug micro infusion through a tattoo machine.
Fig. 7: shows a side view in transparency of the lower portion of a tip used in drug micro infusion through a tattoo machine.
Fig. 8: shows a detailed view of the application in drug micro infusion on the skin through a tattoo machine.

### DETAILED DESCRIPTION OF THE INVENTION

According to the above-mentioned drawings, the present invention relates to gold-plated needles (1a) for injecting drugs into the eyeball (8) and/or the skin (9) by means of tattoo machines to make eye and/or skin disease treatment more effective, more efficient and safer.

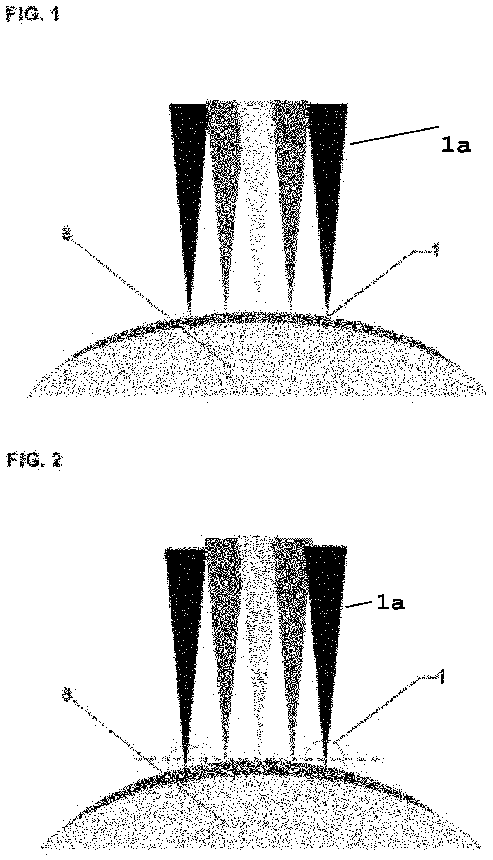

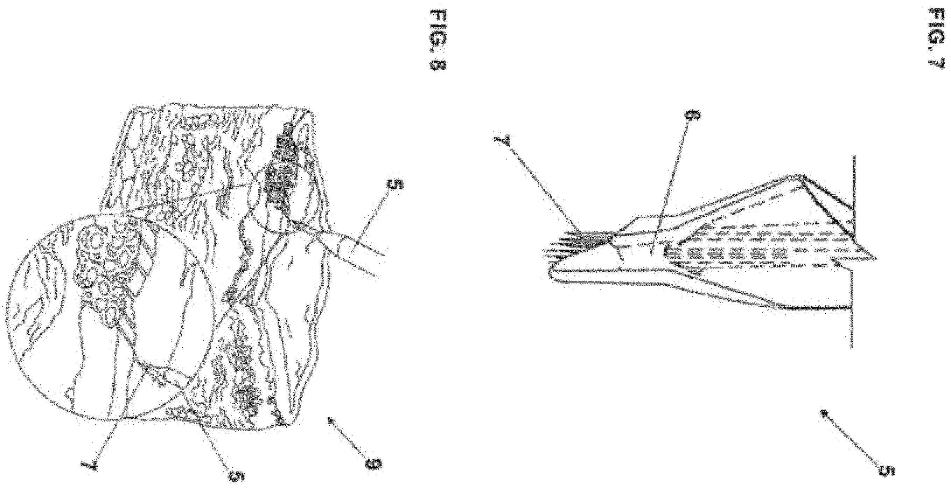

The principle of the invention is very similar to ancient tattoo techniques. In a drug-loaded cartridge (6), with the drug strength being chosen by a physician, the needles (1a), in a back and forth movement, deliver the drug to the corneal stroma (8) and/or eye structures (8) and/or the skin (9). It should be pointed out that, because of the round anatomical structure, the needles (1a), as a whole, should be arranged so that the tips (1) are aligned with the curvature of the eye (8) and/or the skin (9), as shown in Figures 1, 2 and 8.

Drug is delivered by means of an adapted tattoo machine which, instead of ink, uses specific drugs, the tattoo machine has a disposable single-piece tip (5) and a drug container (6) with a mouthpiece projected outwardly and deeply with variable dosing holes, positioned approximately mid-height of the tip (5) and which discharges the drug by shearing onto oscillating needle bundles (7).

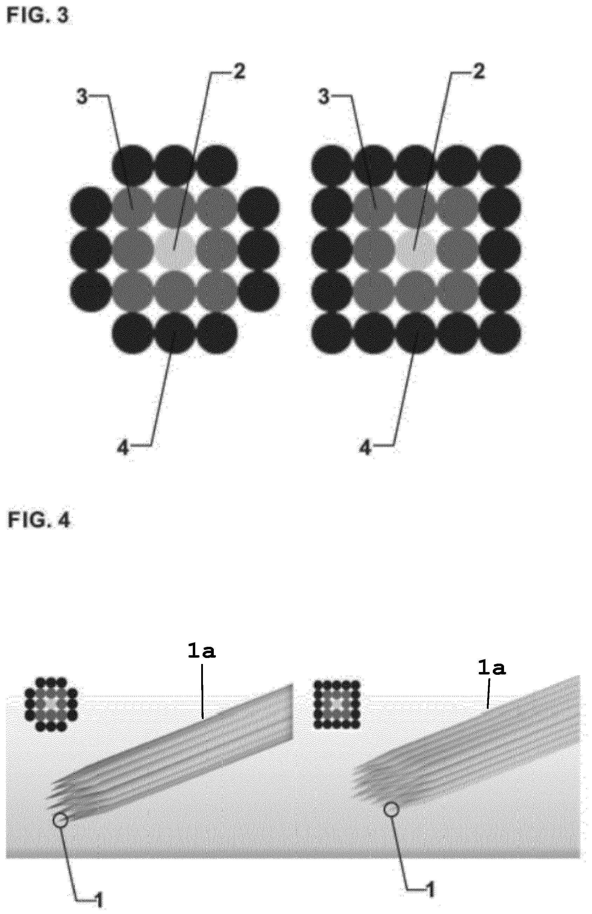

According to Figure 3, the needles (1a) are arranged in a group, the central needle (2) being more retracted, the adjacent needles (3) less retracted and the externally arranged needles (4) are more exposed, so that they align with the curvature of the eye (8).

The needles (1a) are plated with gold for the following reasons:
Gold is less likely to cause allergic reactions in the area to be treated;
Gold has bactericidal properties; and
Gold plating makes the surface of the needle smoother.

The invention is susceptible to variations that may occur in needle morphology (number of needles (1a) diameter and adjustments in tip curvature). Certainly, when the present invention is put into practice, modifications can be made with respect to certain details of construction and shape, without departing from the scope that are clearly substantiated in the claim, being understood that the terminology employed was not intended to be limiting.

This is a descriptive report of a completely new concept which, as it can be seen from the analyzes carried out, shows new technical and functional characteristics.

## Claims

1. Needle improvements for drug delivery using tattoo machines, consisting of needles for injecting drugs into the eyeball and/or the skin, by means of a tattoo machine that comprises a disposable single-piece tip (5) having a drug container (6), with a mouthpiece projected outwardly and deeply with variable dosing holes positioned approximately mid-height of the tip (5) and which discharges the drug by shearing onto oscillating needle bundles (7); the improvements comprising:
- a cartridge (6) loaded with a specific drug delivered from the tattoo machine; and
- needles (1a) comprising tips (1), the needles (1a) being arranged so that the tips (1) are aligned with the curvature of the eye (8) and/or the skin (9), the needles (1a) in the tattoo machine are movable in a back-and-forth movement in order to deliver the drug to the corneal stroma (8) and/or eye structures (8) and/or the skin (9), being **characterized in that** the needles (1a) are arranged in
a group comprising:
- a central needle (2) more retracted,
- adjacent needles (3) less retracted, and
- externally arranged needles (4) that are more exposed that the central (2) and adjacent (3) needles so that they align with the curvature of the eye (8) and/or the skin, wherein all the needles (1a) are plated in gold.

## Patentansprüche

1. Nadelverbesserungen für die Verabreichung von Arzneimitteln unter Verwendung von Tätowiermaschinen, bestehend aus Nadeln zum Injizieren von Arzneimitteln in den Augapfel und/oder die Haut mittels einer Tätowiermaschine, die eine einteilige Einwegspitze (5) mit einem Arzneimittelbehälter (6) umfasst, mit einem nach außen und tief vorstehenden Mundstück mit variablen Dosierlöchern, die etwa auf halber Höhe der Spitze (5) angeordnet sind, und die das Arzneimittel durch Scherung auf oszillierende Nadelbündel (7) abgibt; wobei die Verbesserungen umfassen:
- eine Patrone (6), die mit einem spezifischen Medikament geladen ist, das von der Tätowiermaschine abgegeben wird; und
- Nadeln (1a) mit Spitzen (1), wobei die Nadeln (1a) so angeordnet sind, dass die Spitzen (1) auf die Krümmung des Auges (8) und/oder der Haut (9) ausgerichtet sind, wobei die Nadeln (1a) in der Tätowiermaschine in einer Hin- und Herbewegung beweglich sind, um das Medikament an das Hornhautstroma (8) und/oder die Augenstrukturen (8) und/oder die Haut (9) abzugeben,
**dadurch gekennzeichnet, dass** die Nadeln (1a) in einer Gruppe angeordnet sind, die umfasst:
- eine zentrale Nadel (2), die stärker zurückgezogen ist,
- angrenzende Nadeln (3), die weniger zurückgezogen sind, und
- außen angeordnete Nadeln (4), die stärker exponiert sind als die zentrale (2) und die angrenzenden (3) Nadeln, so dass sie sich an die Krümmung des Auges (8) und/oder der Haut anpassen,
wobei alle Nadeln (1a) mit Gold beschichtet sind.

## Revendications

1. Améliorations d'aiguilles pour l'administration de médicaments en utilisant des machines de tatouage, consistant en des aiguilles pour injecter des médicaments dans le globe oculaire et/ou la peau, au moyen d'une machine de tatouage qui comprend une pointe monobloc jetable (5) ayant un contenant de médicament (6), avec un embout buccal projeté vers l'extérieur et profondément avec des trous de dosage variables positionnés approximativement à mi-hauteur de la pointe (5) et qui décharge le médicament par cisaillement sur des faisceaux d'aiguilles oscillants (7) ; les améliorations comprenant :
- une cartouche (6) chargée d'un médicament spécifique délivré par la machine de tatouage ; et
- des aiguilles (1a) comprenant des pointes (1), les aiguilles (1a) étant agencées de manière à ce que les pointes (1) soient alignées avec la courbure de l'oeil (8) et/ou de la peau (9), les aiguilles (1a) dans la machine de tatouage sont mobiles dans un déplacement en va-et-vient afin de délivrer le médicament au stroma cornéen (8) et/ou aux structures oculaires (8) et/ou à la peau (9),
étant **caractérisées en ce que** les aiguilles (1a) sont agencées en un groupe comprenant :
- une aiguille centrale (2) plus rétractée,
- des aiguilles adjacentes (3) moins rétractées, et
- des aiguilles (4) agencées à l'extérieur qui sont plus exposées que les aiguilles centrale (2) et adjacentes (3) de sorte qu'elles s'alignent avec la courbure de l'oeil (8) et/ou de la peau,
toutes les aiguilles (1a) étant plaquées en or.
